# EUROPEAN PATENT SPECIFICATION

(11) **EP 3 643 236 B1**
(45) Date of publication and mention of the grant of the patent: **01.11.2023**
(21) Application number: 18202381.2
(22) Date of filing: 24.10.2018
(51) Int. Cl.: A61B 5/145, F28D 15/00, F28D 15/02

(54) **AN APPARATUS FOR SENSING BIOMETRIC PARAMETERS**
VORRICHTUNG ZUM ERFASSEN BIOMETRISCHER PARAMETER
APPAREIL DE DÉTECTION DE PARAMÈTRES BIOMÉTRIQUES

(43) Date of publication of application: 29.04.2020
(73) Proprietor: Nokia Technologies Oy, 02610 Espoo (FI)
(72) Inventor: Akshat, Agarwal, Dublin 15 (IE); O'Connell, Diarmuid, Co. Kildare (IE)
(74) Representative: Swindell & Pearson Limited

(56) References cited:
- EP-A1- 3 385 708
- US-A- 5 140 985
- US-A- 5 269 369
- US-A1- 2016 270 700
- US-B2- 9 652 005
- DAVOUD JAFARI ET AL: "Metal 3D-printed wick structures for heat pipe application: Capillary performance analysis", APPLIED THERMAL ENGINEERING, vol. 143, 1 October 2018 (2018-10-01), pages 403-414, XP055578443, GB ISSN: 1359-4311, DOI: 10.1016/j.applthermaleng.2018.07.111

## Description

### TECHNOLOGICAL FIELD

Examples of the present disclosure relate to an apparatus for sensing biometric parameters. Some relate to an apparatus for sensing biometric parameters from a subject's sweat.

### BACKGROUND

The analysis of a subject's sweat can provide information about the physical condition of the subject. Therefore it is useful to provide apparatus that are configured to sense biometric parameters from a subject's sweat.

US 5140985 relates to a non-invasive blood glucose device. The device uses heat from a battery and an insulation band to generate perspiration. A wick moves the perspiration past the sensors.

US 5 269 369 relates to a temperature regulation system for the human body using heat pipes to provide heating or cooling by redistributing body heat.

US 9652 005 relates to a smart watch with a heat transfer component for transferring heat from the smart watch enclosure to a wrist band so that the heat can be dissipated through the wrist band.

Davoud Jafari et al "metal 3D printed wick structures for heat pipe application" ( applied thermal engineering, Vol. 143, 1 October 2018 pages 403-414 relates to metal 3D-printed wick structures for heat pipe applications.

US 2016/270700 relates to a wearable apparatus with a stretch sensor.

### BRIEF SUMMARY

The invention is as defined in the apparatus claim 1 and in the method claim 11.

The heat transfer means may be formed using an additive manufacturing process. The first location may be a location proximate to one or more electronic components.

The one or more electronic components may comprise at least one processor.

The first location may be a location proximate to a region of the subject's body that has a higher temperature than a region in which the one or more sensors are located. The first location may be proximate to the subject's armpit or groin.

The one or more sensors may be configured to be positioned adjacent to a subject's skin.

The one or more sensors may be configured to sense one or more of; sweat composition, sweat rate.

According to various, but not necessarily all, examples of the disclosure there is provided a wearable device comprising one or more apparatus as claimed in any claim.

The wearable device may comprise one or more sweat transfer structures. According to various, but not necessarily all, examples of the disclosure there is provided a method of forming an apparatus as claimed in the claims.

The heat transfer means may be formed using an additive manufacturing process. At least part of the apparatus may be formed on fabric which forms part of a wearable electronic device.

At least part of the apparatus may be formed on a substrate and transferred to fabric which forms part of a wearable electronic device.

According to various, but not necessarily all, examples of the disclosure there is provided a sweat transfer structure comprising: at least one absorption region; at least one evaporation region; and a transport region extending between the at least one absorption region and the at least one evaporation region wherein the transport region comprises a wick structure which is configured to transport sweat from the at least one absorption region to the at least one evaporation region.

The sweat transfer structure may comprise a container configured to collect sweat wherein the container is positioned adjacent to the evaporation region.

The sweat transfer structure may comprise one or more sensors configured to sense one or more parameters from the sweat. The one or more sensors may be configured to sense the presence of one or more analytes within the sweat.

An impermeable coating may be provided over the wick structure in the transport region.

### BRIEF DESCRIPTION

Some example embodiments will now be described with reference to the accompanying drawings in which:
Fig. 1 illustrates an example apparatus;
Fig. 2 illustrates an example wearable electronic device;
Fig. 3 illustrates an example wearable electronic device;
Fig. 4 illustrates an example heat pipe; and
Fig. 5 illustrates an example method; and
Fig. 6 illustrates an example sweat transfer structure.

### DETAILED DESCRIPTION

Examples of the disclosure relate to an apparatus 101 for sensing biometric parameters. The apparatus 101 may comprise one or more sensors 103 configured to sense one or more biometric parameters from a subject's sweat. The apparatus 101 comprises heat transfer means 105 configured to transfer heat from a first location to a location proximate to the one or more sensors 103. The heat transfer means 105 may therefore provide localized heating within the region of the one or more sensors 103. This localized heating may increase the generation of sweat in the region proximate the sensors 103 and so may improve the efficacy of the one or more sensors 103.

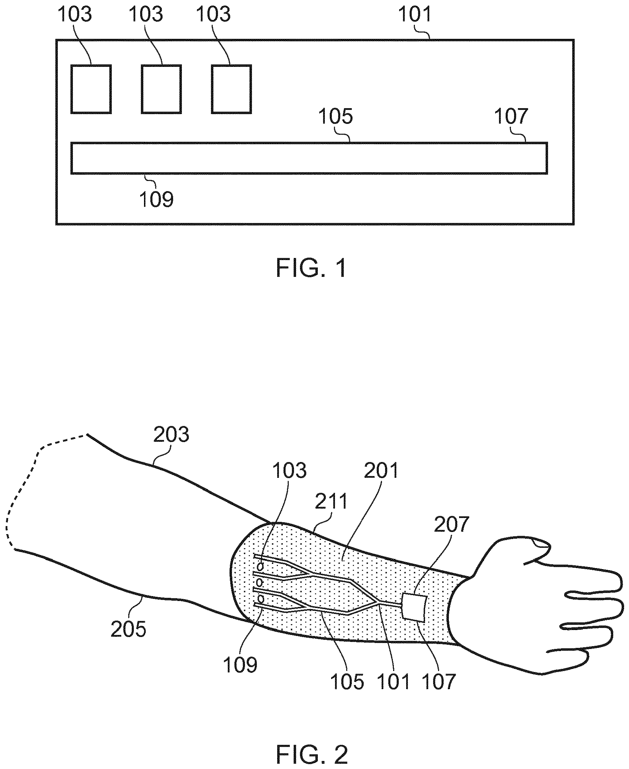

Fig. 1 illustrates an apparatus 101 according to examples of the disclosure. The apparatus 101 comprises a plurality of sensors 103 and a heat transfer means 105.

The sensors 103 may comprise any means which may be configured to sense the sweat of a subject and provide an output indicative of the sensed sweat. In some examples the sensors 103 could be configured to sense the presence of one or more analytes within the sweat, for example the sensors 103 could be configured to detect the presence of a particular chemical within the sweat. In some examples the sensors 103 could be configured to detect the sweat rate or any other suitable parameter.

The sensors 103 may comprise any material which may be configured to be sensitive to the sweat and/or an analyte within the sweat. The sensors 103 could comprise a material which has an electrical property that changes in the presence of sweat or the presence of an analyte within the sweat.

The sensors 103 may have any suitable transduction mechanism to convert the sensed sweat or analyte into an output signal. In some examples the sensors 103 may have a capacitive or a conductive transduction mechanism. If the sensors 103 have a capacitive transduction mechanism then the presence of the sweat and/or analyte may change the permittivity of the material in the sensor 103. The capacitance of the sensor 103 may have a known variation as a function of the concentration of the sweat and/or analyte. In such examples the sensors 103 may comprise a material such as polymeric material or any other suitable material.

If the sensors 103 have a conductive transduction mechanism then the presence of the sweat and/or analyte may change the conductivity of the material. The conductance of the sensor 103 may have a known variation as a function of the concentration of the sweat and/or analyte. In such examples the sensors 103 may comprise a material such as graphene oxide, graphene, functionalised graphene materials, boron nitride, molybdenite or any other suitable material.

The material which is chosen for use within the sensors 103 may depend on the analytes within the sweat that are to be detected. In some examples the same material may be used for each of the plurality of sensors 103. In some embodiments different materials may be used for different sensors 103. The use of different materials may enable different biometric parameters to be monitored.

In some examples the sensors 103 could be configured to detect biometric parameters from the subject's sweat which can be used in the diagnosis of diseases. For instance chloride concentration within sweat may be used to detect cystic fibrosis while potassium concentration within sweat may be used in the early diagnosis of cystic fibrosis and during treatment of cystic fibrosis. The concentrations of chemicals such as cadmium and lead within a subject's sweat could also be used in the diagnosis of some kidney diseases. Sweat rates and sweat composition may also be used to assist in the detection of diabetes. The presence of analytes within sweat could also be used as an indication of some types of cancer. For instance chemical compounds such as dermcidin (DCD) can be detected in sweat and are involved in tumorigenesis by promoting cell growth and survival in breast carcinomas. Prolactin inducible protein (PIP) can also be detected in sweat and is overexpressed in metastatic breast and prostate cancer. The chemical analysis of sweat may also be used to discriminate between metabolomics of patients with and without lung cancer. Other chemicals and analytes may be detected in other examples of the disclosure.

In the example apparatus 101 shown in Fig. 1 the apparatus 101 comprises three sensors 103. It is to be appreciated that the apparatus 101 could comprise any suitable number of sensors 103. In some examples the apparatus 101 could comprise only one sensor 103. In some examples the apparatus 101 could comprise a plurality of sensors 103 which could be located in different positions. For instance the sensors 103 could be positioned so that different sensors 103 are proximate to different parts of the subject's body.

The sensors 103 are positioned within the apparatus 101 so that when the apparatus 101 is in use the sensors 103 are positioned adjacent to the skin of a subject. The sensors 103 may be positioned adjacent to the skin of a subject so that sweat from the subject can come into direct contact with the one or more sensors 103.

The apparatus 101 also comprises heat transfer means 105. The heat transfer means 105 enable heat to be transferred from a first location 107 to a second location 109.

The heat transfer means 105 may comprise any suitable thermally conductive material. The thermally conductive material enables heat from the first location to be transferred into the heat transfer means 105 and transferred to the second location 109. In some examples the heat transfer means 105 may comprise a metal such as copper or any other suitable material. In some examples different parts of the heat transfer means 105 may be formed from different materials. For instance a thermally conductive material could be used at the ends of the heat transfer means 105 where heat is to be exchanged but a thermally insulating material could be provided between the ends of the heat transfer means 105 to prevent the heat loss between the first location 107 and the second location 109.

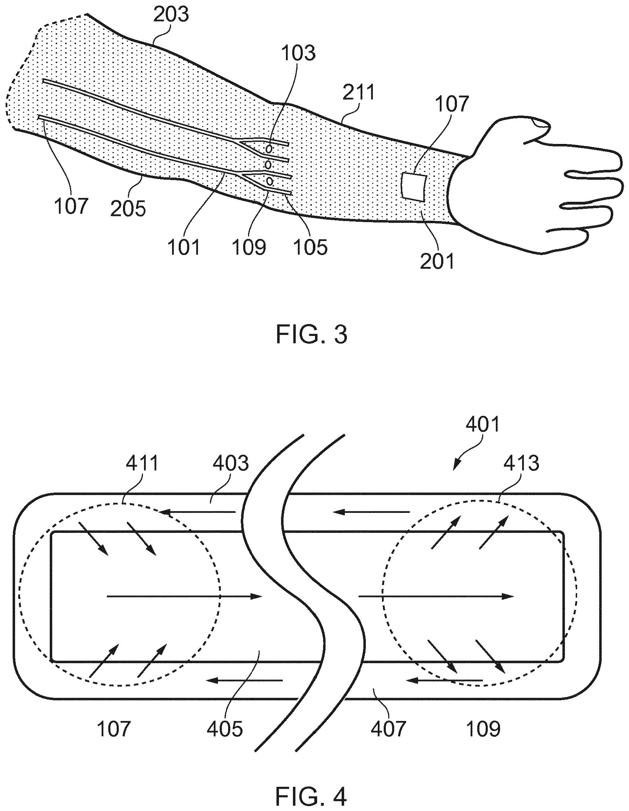

In the claimed invention the heat transfer means 105 comprise a heat pipe. An example of a heat pipe that may be used in examples of the disclosure is shown in Fig. 4. It is to be appreciated that other configurations of heat pipes could also be used in some examples of the disclosure. In other examples the heat transfer means 105 could comprise a conductive trace or any other suitable means.

In some examples the heat transfer means 105 may be flexible and/or comprise flexible portions. The heat transfer means 105 may be flexible so that the heat transfer means 105 can be easily deformed by a subject. This may enable the apparatus 101 to be integrated within a wearable electronic device. The flexibility of the heat transfer means 105 may enable a subject who is wearing the wearable device to move freely while wearing the wearable electronic device.

The heat transfer means 105 is configured to enable heat to be transferred from a first location 107 to a second location 109. The first location 107 is a region that has a higher temperature than the second location 109. An insulating material may be provided around the heat transfer means 105 in the sections between the first location 107 and the second location 109 so as to prevent heat being dissipated at locations other than the second location 109.

The heat transfer means 105 may be small to enable the heat transfer means 105 to be embedded within a wearable electronic device. In some examples the heat transfer means 105 could have a width within a range between tens of micrometers o hundreds of micrometers. The heat transfer means 105 may have a flat, or substantially flat, profile so that the heat transfer means 105 can be embedded within a wearable electronic device 201. The length of the heat transfer means 105 may depend on the relative location of the first location 107 and the locations of the one or more sensors 103.

The first location 107 may be a location which is proximate to, or adjacent to, one or more electronic components. The electronic components could comprise any components which generate heat during use. The electronic components could comprise high powered electronic components. For example, the electronic components could comprise one or more processors, displays or any other suitable components. The electronic components could be part of the apparatus 101 or could be provided separately. Where the apparatus 101 is provided within a wearable electronic device the electronic components could be provided in a different location within the wearable electronic device to the one or more sensors 103.

In some examples the first location 107 may be a location proximate to a region of the subject's body that has a higher temperature than the region in which the one or more sensors 103 are located. For instance the first location 107 could be proximate to a subject's armpit or groin area. This could have a higher temperature than a region such as the subject's arm or back which may be the region where the one or more sensors 103 are located.

The heat transfer means 105 provides localised heating in the second location 109. The second location 109 is a location proximate to the one or more sensors 103. The second location 109 is proximate to the one or more sensors 103 so that the heating caused by the heat transfer means 105 causes more sweat to be generated in the region around the one or more sensors 103. This increases the volume of sweat that is available to be analysed by the one or more sensors 103. This may increase the efficacy of the one or more sensors 103

The apparatus 101 may be provided within a wearable electronic device 201. An example of a wearable electronic device 201 is shown in Fig. 2.

In the example of Fig. 2 the wearable electronic device 201 comprises a sleeve 211 which is worn on the arm 203 of a subject 205. In the example of Fig. 2 the sleeve 211 is worn around the subject's lower arm 203 so that the sleeve 211 extends between the subject's wrist and the subject's elbow.

The sleeve 211 could be formed from fabric. In some examples the sleeve 211 could be formed from a stretchable fabric so that the sleeve 211 fits tightly around the subject's arm 203. Other materials may be used in other examples of the disclosure.

In the example of Fig. 2 the wearable electronic device 201 comprises an apparatus 101 and one or more electronic components 207. The apparatus 101 may comprise a plurality of sensors 103 and a heat transfer means 105 which may be as described in relation to Fig. 1. Corresponding reference numerals are used for corresponding features.

The electronic component 207 could be any electronic component which is used to enable one or more functions of the wearable electronic device 201. In the example of Fig. 2 the electronic component 207 is a high powered electronic component which generates heat during use. For example the electronic component 207 could comprise a processor, a display or any other suitable component.

The electronic component 207 is located at a first position 107 within the wearable electronic device 201. In the example wearable electronic device 201 of Fig. 2 the electronic component 207 is located so that when the wearable electronic device 201 is being worn by the subject the electronic component 207 is positioned adjacent to the subject's wrist. The electronic component 207 could be positioned in other locations in other examples of the disclosure.

The plurality of sensors 103 are located at a second position 109 within the wearable electronic device 201. In the example wearable electronic device 201 of Fig. 2 the plurality of sensors 103 are located within the wearable electronic device 201 so that, when the wearable electronic device 201 is being worn by the subject 205 the plurality of sensors 103 are positioned close to the subject's elbow. The sensors 103 may be positioned in other locations in other examples of the disclosure.

The heat transfer means 105 extends between the first location 107 which is proximate to the electronic component 107 and the second location 109 which is proximate to the one or more sensors 103. This diverts heat from the area around the electronic component 207 to the area around the one or more sensors 103. This provides cooling the in area around the electronic components and prevents over heating in this area. This also provides localised heating in the area around the sensors 103 so as to increase the generation of sweat and improve the efficacy of the sensors 103.

Fig. 3 illustrates another example wearable electronic device 201. The wearable electronic device 201 shown in Fig. 3 is similar to the wearable electronic device 201 as shown in Fig. 2 in that the wearable electronic device 201 comprises a sleeve 211 which comprises an apparatus 101 and one or more electronic components 207.

In the example of Fig. 3 the sleeve 211 of the wearable electronic device 201 is worn around the whole of the subject's arm 203 so that the sleeve 211 extends between the subject's wrist and the subject's armpit.

In the example of Fig. 3 the heat transfer means 105 of the apparatus 101 extend from a first location 107 which is proximate to the subject's armpit to a second location 109 which is proximate to the sensors 103 of the apparatus 101. The armpit has a higher natural temperature than the section of the arm 203 in which the sensors 103 are positioned so that heat is transferred, via the heat transfer means 105, from the warmer area to the cooler area. This provides cooling the in area proximate to the subject's armpit and may reduce sweating in this area. This also provides localised heating in the area around the sensors 103 so as to increase the generation of sweat and improve the efficacy of the sensors 103.

In the example of Fig. 3 the heat transfer means 105 extends over the subject's elbow. In this example the heat transfer means 105 may be flexible to enable the subject to bend their arm 203 at the elbow. This ensures that the wearable electronic device 201 is comfortable for the subject 205 to wear.

In the examples of Figs. 2 and 3 the heat transfer means 105 is configured to transfer heat either from a location proximate to the electronic component 207 or a location proximate to a warmer part of the subject's body. It is to be appreciated that in other examples the heat transfer means 105 could transfer heat from a plurality of different locations. For instance the heat transfer means 105 could transfer heat from both a location proximate to an electronic component 207 and a location proximate to a warmer part of the subject's body, or could transfer heat from a plurality of locations proximate to electronic components 207, or could transfer heat from a plurality of locations proximate to warmer parts of the subject's body. In such examples the heat transfer means 105 could be configured to have a branch structure so that it can transfer heat from the plurality of different first locations 107.

Also in the examples of Figs. 2 and 3 the sensors 103 are located in a single location close to the subject's elbow. It is to be appreciated that the sensors 103 could be positioned in different locations in other examples of the disclosure. For instance the sensors 103 could be positioned adjacent to the subject's torso or leg or any other suitable part of their body. In some examples the sensors 103 could be located in a plurality of different locations. In such examples the heat transfer means 105 could be configured to have a branched structure so that it can transfer heat to the plurality of different locations.

In some examples the wearable electronic device 201 may comprise one or more electronic components which may be configured to communicate data obtained from the one or more sensors 103 with or without local storage of the data in a memory within the wearable electronic device 201 and with or without local processing of the data by circuitry or processors within the wearable electronic device 201.

The data may, for example, be measurement data obtained from the one or more sensors 103 or data produced by the processing of measurement data from the one or more sensors 103. The data produced by the processing of the measurement data could provide an indication of the concentration of one or more analytes within the subject's sweat, the sweat rate, or any other suitable parameter.

The data may be stored in processed or unprocessed formats remotely at one or more devices.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The wearable electronic device 201 may comprise a communications interface such as, for example, a radio transceiver for communication of data.

The wearable electronic device 201 may be part of a distributed network such as the Internet of Things.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output. The output may be communicated to the wearable electronic device 201 where it may produce an output sensible to the subject such as an audio output, visual output or haptic output.

Fig. 4 schematically illustrates a cross section example heat pipe 401 that could be used as a heat transfer means 105 in the present invention. The heat pipe 401 comprises three layers an upper layer 403, a gas channel 405 and a lower layer 407. The upper layer 403 and the lower layer 407 provide a wick structure around the gas channel 405. The wick structure comprises a plurality of capillary channels that enable liquid to be transported along the upper layer 403 and the lower layer 407.

A working fluid is provided within the heat pipe 401. The working fluid could be water or any other suitable fluid. When the heat pipe 401 is in use the working fluid circulates through the gas channel 405 and the upper layer 403 and the lower layer 407 so as transfer heat from the first location 107 to the second location 109. When the working fluid is in the gas channel 405 the working fluid is in a gas phase and when the working fluid is in the upper layer 403 or the lower layer 407 the working fluid is in a liquid phase.

A first end of the heat pipe 401 provides an evaporator region 411 in a first location 107. The apparatus 101 may be formed so that in use the evaporator region 411 may be located in a high temperature location. The high temperature location could be proximate to an electronic component 107 or proximate to a warm part of the subject's body or any other suitable location. The first location 107 could be a high temperature location in that it is warmer than other parts of the apparatus 101 or wearable electronic device 201.

A second end of the heat pipe 401 provides a condenser region 413 in a second location 109. The apparatus 101 may be formed so that in use the condenser region 413 may be located proximate to the one or more sensors 103 of the apparatus 101. The second location 109 may be a low temperature location in that it has a lower temperature than the first location 107.

At the evaporator region 411, the heat from the first location 107 causes the working fluid to evaporate and change phase from a liquid to a gas. The working fluid in the gas phase travels from the evaporator region 411 to the condenser region 413. At the condenser region 413, the comparatively cooler temperature causes the working fluid to condense and change phase from a gas to a liquid. As result, heat is moved from the evaporator region 411 in the first location 107 to the condenser region 413 in the second location 109.

At the condenser region 413, the working fluid condenses back into a liquid phase and travels back to the evaporator region 411 through capillary action of the wick structure in the upper layer 403 and the lower layer 407. Once the liquid phase working fluid has reached the evaporator region 411 again the heat at the evaporator region 411 will change the working fluid back into the gas phase. The cycle of the working fluid changing phase repeats so as to drive the working fluid in the gas phase and the heat from the evaporator region 411 to the condenser region 413.

In some examples the section of the heat pipe 401 between the evaporator region 411 and the condenser region 413 may be insulated. The insulation may help to prevent heat being lost through the heat pipe 401 to locations between the first location 107 and the second location 109.

In the example of Fig. 4 the heat pipe 401 comprises a first end and a second end where the evaporator region 411 is located at the first end and the condenser region 413 is located at the second end. In other examples the heat pipe 401 could comprise multiple branches. For instance the wearable electronic device 201 could comprise a plurality of different evaporator regions 411 which could be located at different first locations 107 within a wearable electronic device 201. As an example the wearable electronic device 201 could comprise a plurality of electronic components 207 and the heat pipe 401 could comprise a plurality of different evaporator regions 411 which may be located in proximity to the different electronic components 107.

In some examples the evaporator region 411 of the heat pipe 401 may be in the proximity of, or in direct contact with, the electronic component 107. This may help to keep the thermal resistance between the heat pipe 401 and the electronic component 107 as low as possible. For instance, the heat pipe 106 and the electronic component 107 may be encapsulated whilst in contact with one another.

In some examples the wearable electronic device 201 could comprise a plurality of different condenser regions 413 which could be located at different second locations 109 within a wearable electronic device 201. As an example the wearable electronic device 201 could comprise a plurality of different sensors 103 which could be positioned adjacent to different parts of the subject's body. The heat pipe 401 could comprise a plurality of different condenser regions 413 which may be located in proximity to the different sensors 103.

In some examples the condenser region 413 of the heat pipe 401 may be in the proximity of, or in direct contact with, the skin of the subject 205. This may help to keep the thermal resistance between the heat pipe 401 and the subject 205 as low as possible to increase the localised heating of the subject 205 in the area around the sensors 103.

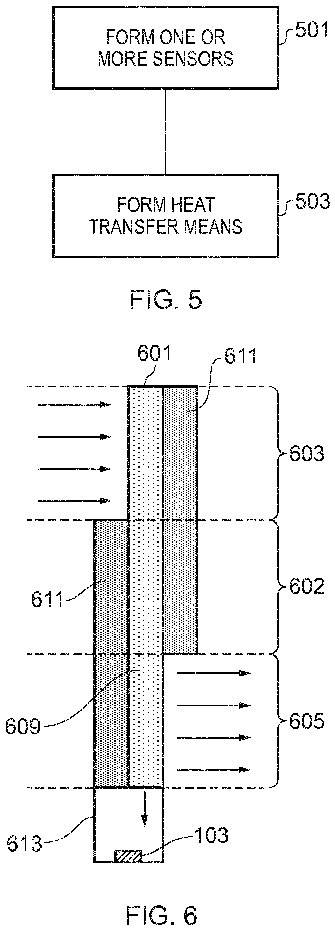

Fig. 5 illustrates an example method that may be used to form apparatus 101 as described above. The method comprises, at block 501 forming one or more sensors 103 where the sensors 103 are configured to sense one or more biometric parameters from a subject's sweat. The sensors 103 could comprise a material which has an electrical property that changes in the presence of sweat or the presence of an analyte within the sweat.

The one or more sensors 103 could be formed using any suitable process. In some examples the one or more sensors 103 could be formed using an additive manufacturing process such as three dimensional printing. In other examples the sensors 103 could be formed using two dimensional printing techniques or any other suitable processes.

At block 503 the method comprises forming heat transfer means 105. The heat transfer means 105 are configured to transfer heat from a first location 107 to a second location 109 which is proximate to the one or more sensors 103. The heat transfer means 105 comprise a heat pipe 401.

The heat transfer means 105 may be formed using an additive manufacturing process such as three dimensional printing. The additive manufacturing process may enable the heat transfer means 105 to be formed from a plurality of different materials. For instance, where the heat transfer means 105 comprises a heat pipe 401 the heat pipe 401 could comprise a thermally conductive material in the evaporator region 411 and the condenser region 413 but could also comprise a thermally insulating material in the region between the evaporator region 411 and the condenser region 413 so as to reduce heat loss.

The use of an additive manufacturing process may also enable a complex heat transfer means 105 to be formed. For example this may enable the heat transfer means 105 to have multiple branches. It may also enable internal structures such as wick structures for enabling capillary action to be performed.

In some examples the apparatus 101 may be formed on fabric where the fabric forms part of a wearable electronic device 201. In such examples the apparatus 101 could be formed directly onto the fabric that forms the wearable electronic device 201 so that the apparatus 101 is integrated within the wearable electronic device 201. In other examples at least part of the apparatus 101 may be formed on a substrate and transferred to fabric which forms part of the wearable electronic device 201. This process may be useful where the fabric of the wearable electronic device 201 is not suitable for use a substrate for printing. For example, if the fabric of the wearable electronic device 201 comprises a stretchable material.

Fig. 6 illustrates an example sweat transfer structure 601. The sweat transfer structure 601 comprises at least one absorption region 603, at least one evaporation region 605 and a transport region 607.

The absorption region 603 comprises a region which is configured to absorb sweat from the surface of the skin of a subject. In the example shown in Fig. 6 the absorption region 603 comprises an exposed wick structure 609. The wick structure 609 is positioned within the absorption region 603 so that when the sweat transfer structure 601 is in use the exposed wick structure 609 is positioned adjacent to the skin of the subject. The exposed wick structure 609 may be positioned adjacent to the skin of a subject so that sweat from the subject can be drawn into the wick structure 609.

In the example of Fig. 6 an impermeable coating 611 is provided overlaying at least part of the wick structure 609 in the absorption region 603. The impermeable coating 611 may be provided on an opposing side of the wick stricture 609 to the exposed area of the wick structure 609. This may ensure that the sweat is retained within the wick structure 609 and does not simply evaporate straight away.

The evaporation region 605 comprises a region which is configured to enable sweat to be evaporated into the atmosphere. In the example shown in Fig. 6 the evaporation region 605 comprises another exposed wick structure 609. The wick structure 609 is positioned within the evaporation region 605 so that when the sweat transfer structure 601 is in use the exposed wick structure 609 is exposed to the atmosphere and is not positioned adjacent to the skin of the subject. The exposure to the atmosphere enables the sweat from the wick structure 609 to be evaporated into the atmosphere.

In the example of Fig. 6 an impermeable coating 611 is provided overlaying at least part of the wick structure 609 in the evaporation region 605. The impermeable coating 611 may be provided on an opposing side of the wick structure 609 to the exposed area of the wick structure 609 so that the impermeable coating 611 is on the same side as the skin of the subject. This may ensure that the sweat is directed towards the atmosphere and not back towards the skin of the subject.

The transport region 607 extends between the at least one absorption region 603 and the at least one evaporation region 605. The transport region 607 comprises a wick structure 609 which is configured to transport sweat from the at least one absorption region 603 to the at least one evaporation region 605 by capillary action. In examples of the disclosure the evaporation of sweat into the atmosphere in the evaporation region 605 causes more sweat to be drawn through the wick structure 609 in the transport region 607 and causes more sweat to be absorbed in the absorption region 603. The capillary action works without the need for any external power source and the wick structure 609 can be configured so that, if needed, the capillary action can work against gravitational forces. This therefore enables sweat to be transferred away from the absorption region to the evaporation region. This could enable sweat to be transferred away from an area which produces a relatively large amount of sweat, such as an armpit or groin, to another location.

In the example of Fig. 6 an impermeable coating 611 is provided over the wick structure 609 in the transport region 607. In the transport region 607 the impermeable coating 611 may cover the wick structure 609 so that the sweat is retained within the wick structure 609 and provided to the evaporation region 605. The impermeable coating 611 may comprise any suitable material which does not allow the sweat to pass through. The impermeable coating 611 may comprise plastics, polymer or any other suitable material.

In the example shown in Fig. 6 the sweat transfer structure 601 also comprises a container 613. The container 613 is provided adjacent to the evaporation region 607 and is configured to collect sweat which is not evaporated into the atmosphere. The use of the container 613 could prevent the sweat from leaking into the fabric of a wearable device which may cause an unwanted odour or staining.

In some examples the sweat transfer structure 601 could comprise one or more sensors 103. The sensors 103 could be configured to sense one or more parameters from the sweat that is passed through the sweat transfer structure 601. For instance the sensors 103 could be configured to sense the presence of one or more analytes within the sweat, the sweat rate or any other suitable parameter. The sensors 103 could be similar to the sensors 103 used in the apparatus 101 shown in Fig. 1. In some examples the sensors 103 could be provided within the container 613 so that the sweat collected within the container comes into contact with the one or more sensors 103. In other examples the sensors 103 could be provided in different locations within the sweat transfer structure 601.

In some examples the sweat transfer structure 601 may be formed using an additive manufacturing process such as three dimensional printing. The additive manufacturing process may enable the sweat transfer structure 601 to be formed from a plurality of different materials. For instance, the wick structure 609 and the impermeable coating 611 could be formed from different materials.

The use of an additive manufacturing process may also enable a complex sweat transfer structure 601 to be formed. For example, this may enable the sweat transfer structure 601 to have multiple branches. This could enable sweat from a plurality of different locations to be transferred to a single container 613 and/or sensor 103. It may also enable directional structures to be formed within the wick structure 609 which may help to control the direction of flow of the sweat within the sweat transfer structure 601.

In some examples the sweat transfer structure 601 may be provided in a wearable electronic device 201 with one or more apparatus 101 such as the apparatus 101 shown in Fig. 1. This could enable additional sweat from other parts of the body to be provided to the one or more sensors 103. This could increase the volume of sweat which is analysed.

In some examples the same sensors 103 could be used to sense sweat from the sweat transfer structure 601 and sweat from skin which is adjacent to the sensors 103. This may enable more sweat to be analysed. In some examples different sensors 103 could be used to sense sweat from the sweat transfer structure 601 and sweat from skin which is adjacent to the sensors 103. This may enable different parameters of the sweat to be analysed. For example, sweat from a subject's armpit may have a different composition compared to sweat from a subject's arm or leg. The use of the different sensors 103 may enable these different compositions to be analysed.

The use of the sweat transfer structure 601 may also provide additional benefits in that it may transport sweat away from the hotter regions of the subject. This may help to reduce staining and odour that could be caused by the sweat. It may also help to cool the subject down and keep the subject more comfortable.

Examples of the disclosure provide an apparatus 101 where unwanted heat is transferred from a first location 107 to a location proximate to one or more sensors 103 to provide localized heating and increase the production of sweat around the sensors 103. This increases the efficacy of the sensors 103.

The apparatus 101 also removes heat from areas where it is undesirable. For instance, in examples where the heat transfer means 105 removes heat from electronic components 207 this provides cooling of the electronic component 207. This may enable the electronic component 207 to run more efficiently and may also prevent overheating of the electronic component 207 which may be uncomfortable for a subject 205. In some examples the heat transfer means 105 may remove heat from warmer areas of the subject's body. In these examples this could aid in cooling of the subject 205 and could help to reduce sweating for the subject 205. This may help to make the subject 205 more comfortable.

The heat transfer means 105 may be configured to transfer heat from any suitable first location 107 to any suitable second location 109. This may enable the sensors 103 to be located in any suitable location. This provides greater design freedom for the location of the sensors 103 as the heat needed to provide localized heating and generate the required amount of sweat can be transferred to wherever it is needed. This may enable the sensors 103 to be positioned in a location which is more comfortable for the subject.

Although embodiments have been described in the preceding paragraphs with reference to various examples, it should be appreciated that modifications to the examples given can be made without departing from the scope of the claims.

## Claims

1. An apparatus (101) comprising:
one or more sensors (103) configured to sense one or more biometric parameters from a subject's sweat; and
at least one heat pipe (401) configured to transfer heat from a first location to a location proximate to the one or more sensors (103).

2. An apparatus (101) as claimed in any preceding claim wherein the at least one heat pipe (401) is formed using an additive manufacturing process.

3. An apparatus (101) as claimed in any preceding claim wherein the first location is a location proximate to one or more electronic components.

4. An apparatus (101) as claimed in claim 3 wherein the one or more electronic components comprises at least one processor.

5. An apparatus (101) as claimed in any preceding claim wherein the first location is a location proximate to a region of the subject's body that has a higher temperature than a region in which the one or more sensors (103) are located.

6. An apparatus (101) as claimed in claim 5 wherein the first location is proximate to the subject's armpit or groin.

7. An apparatus (101) as claimed in any preceding claim wherein the one or more sensors (103) are configured to be positioned adjacent to a subject's skin.

8. An apparatus (101) as claimed in any preceding claim wherein the one or more sensors are configured to sense one or more of; sweat composition, sweat rate.

9. A wearable device (201) comprising one or more apparatus as claimed in any preceding claim.

10. A wearable device (201) as claimed in claim 9 comprising one or more sweat transfer structures.

11. A method of forming an apparatus (101) comprising:
one or more sensors (103) configured to sense one or more biometric parameters from a subject's sweat; and
at least one heat pipe (401) configured to transfer heat from a first location to a location proximate to the one or more sensors (103).

12. A method as claimed in claim 11 wherein the at least one heat pipe is formed using an additive manufacturing process.

13. A method as claimed in any of claim 11 to 12 wherein at least part of the apparatus (101) is formed on fabric which forms part of a wearable electronic device.

14. A method as claimed in any of claims 11 to 13 wherein at least part of the apparatus (101) is formed on a substrate and transferred to fabric which forms part of a wearable electronic device (201).

## Patentansprüche

1. Einrichtung (101), die Folgendes umfasst:
einen oder mehrere Sensoren (103), die dazu ausgelegt sind, einen oder mehrere biometrische Parameter vom Schweiß einer Person zu erfassen; und
mindestens eine Wärmeleitung (401), die dazu ausgelegt ist, Wärme von einem ersten Ort zu einem Ort in der Nähe des einen oder der mehreren Sensoren (103) zu übertragen.

2. Einrichtung (101) nach einem der vorhergehenden Ansprüche, wobei die mindestens eine Wärmeleitung (401) unter Verwendung eines additiven Fertigungsprozesses gebildet wird.

3. Einrichtung (101) nach einem der vorhergehenden Ansprüche, wobei der erste Ort ein Ort in der Nähe von einer oder mehreren elektronischen Komponenten ist.

4. Einrichtung (101) nach Anspruch 3, wobei die eine oder die mehreren elektronischen Komponenten mindestens einen Prozessor umfassen.

5. Einrichtung (101) nach einem der vorhergehenden Ansprüche, wobei der erste Ort ein Ort in der Nähe einer Region des Körpers der Person ist, die eine höhere Temperatur aufweist als eine Region, in der sich der eine oder die mehreren Sensoren (103) befinden.

6. Einrichtung (101) nach Anspruch 5, wobei der erste Ort in der Nähe der Achselhöhle oder der Leistenbeuge der Person liegt.

7. Einrichtung (101) nach einem der vorhergehenden Ansprüche, wobei der eine oder die mehreren Sensoren (103) dazu ausgelegt sind, neben der Haut einer Person positioniert zu sein.

8. Einrichtung (101) nach einem der vorhergehenden Ansprüche, wobei der eine oder die mehreren Sensoren dazu ausgelegt sind, eines oder mehreres von einer Schweißzusammensetzung, einer Schwitzrate zu erfassen.

9. Tragbare Vorrichtung (201), die eine oder mehrere Einrichtungen nach einem der vorhergehenden Ansprüche umfasst.

10. Tragbare Vorrichtung (201) nach Anspruch 9, die eine oder mehrere Schweißübertragungsstrukturen umfasst.

11. Verfahren zum Bilden einer Einrichtung (101), das Folgendes umfasst:
einen oder mehrere Sensoren (103), die dazu ausgelegt sind, einen oder mehrere biometrische Parameter vom Schweiß einer Person zu erfassen; und
mindestens eine Wärmeleitung (401), die dazu ausgelegt ist, Wärme von einem ersten Ort zu einem Ort in der Nähe des einen oder der mehreren Sensoren (103) zu übertragen.

12. Verfahren nach Anspruch 11, wobei die mindestens eine Wärmeleitung unter Verwendung eines additiven Fertigungsprozesses gebildet wird.

13. Verfahren nach einem der Ansprüche 11 bis 12, wobei mindestens ein Teil der Einrichtung (101) auf Stoff gebildet ist, der einen Teil einer tragbaren elektronischen Vorrichtung bildet.

14. Verfahren nach einem der Ansprüche 11 bis 13, wobei mindestens ein Teil der Einrichtung (101) auf einem Substrat gebildet ist und zu einem Stoff übertragen wird, der einen Teil einer tragbaren elektronischen Vorrichtung (201) bildet.

## Revendications

1. Appareil (101) comprenant :
un ou plusieurs capteurs (103) configurés pour détecter un ou plusieurs paramètres biométriques à partir de la sueur d'un sujet ; et
au moins un caloduc (401) configuré pour transférer la chaleur d'un premier emplacement à un emplacement proche des un ou plusieurs capteurs (103).

2. Appareil (101) selon l'une des revendications précédentes, dans lequel l'au moins un caloduc (401) est formé à l'aide d'un processus de fabrication additive.

3. Appareil (101) selon l'une des revendications précédentes, dans lequel le premier emplacement est un emplacement proche d'un ou plusieurs composants électroniques.

4. Appareil (101) selon la revendication 3, dans lequel les ou plusieurs composants électroniques comprennent au moins un processeur.

5. Appareil (101) selon l'une des revendications précédentes, dans lequel le premier emplacement est un emplacement proche d'une région du corps du sujet qui a une température plus élevée qu'une région dans laquelle les un ou plusieurs capteurs (103) sont situés.

6. Appareil (101) selon la revendication 5, dans lequel le premier emplacement est proche de l'aisselle ou de l'aine du sujet.

7. Appareil (101) selon l'une des revendications précédentes, dans lequel les un ou plusieurs capteurs (103) sont configurés pour être positionnés de manière adjacente à la peau d'un sujet.

8. Appareil (101) selon l'une des revendications précédentes, dans lequel les un ou plusieurs capteurs sont configurés pour détecter un ou plusieurs parmi : une composition de la sueur, un taux de transpiration.

9. Dispositif portable (201) comprenant un ou plusieurs appareils selon l'une des revendications précédentes.

10. Dispositif portable (201) selon la revendication 9, comprenant une ou plusieurs structures de transfert de la sueur.

11. Procédé de formation d'un appareil (101) comprenant :
un ou plusieurs capteurs (103) configurés pour détecter un ou plusieurs paramètres biométriques à partir de la sueur d'un sujet ; et
au moins un caloduc (401) configuré pour transférer la chaleur d'un premier emplacement à un emplacement proche des un ou plusieurs capteurs (103).

12. Procédé selon la revendication 11, dans lequel l'au moins un caloduc est formé à l'aide d'un processus de fabrication additive.

13. Procédé selon l'une des revendications 11 et 12, dans lequel au moins une partie de l'appareil (101) est formée sur un tissu faisant partie d'un dispositif électronique portable.

14. Procédé selon l'une des revendications 11 à 13, dans lequel au moins une partie de l'appareil (101) est formée sur un substrat et transférée sur un tissu faisant partie d'un dispositif électronique portable (201).
